(19) Europäisches Patentamt
European Patent Office
Office européen des brevets

(11) **EP 2 110 175 A1**

(12) **EUROPÄISCHE PATENTANMELDUNG**

(43) Veröffentlichungstag:
21.10.2009 Patentblatt 2009/43

(21) Anmeldenummer: 08005370.5

(22) Anmeldetag: **20.03.2008**

(51) Int Cl.:
*B01L 7/00* (2006.01)        *H01F 1/36* (2006.01)
*H01F 1/37* (2006.01)        *H05B 6/02* (2006.01)
*H05B 6/10* (2006.01)

(84) Benannte Vertragsstaaten:
AT BE BG CH CY CZ DE DK EE ES FI FR GB GR
HR HU IE IS IT LI LT LU LV MC MT NL NO PL PT
RO SE SI SK TR
Benannte Erstreckungsstaaten:
AL BA MK RS

(71) Anmelder: **Fraunhofer-Gesellschaft zur Förderung der angewandten Forschung e.V.
80686 München (DE)**

(72) Erfinder:
• **Zilch, Christian
04275 Leipzig (DE)**

• **Gerdes, Wilhelm
04275 Leipzig (DE)**
• **Bauer, Johann
64283 Darmstadt (DE)**
• **Holschuh, Karl
64342 Seeheim-Jugenheim (DE)**

(74) Vertreter: **Katzameyer, Michael
v. Bezold & Partner
Patentanwälte
Akademiestrasse 7
80799 München (DE)**

(54) **Verfahren und Vorrichtung zur thermischen Steuerung biologischer und chemischer Reaktionen unter Einsatz von magnetischen Partikeln oder magnetischen Beads und Wechselmagnetfeldern**

(57)     Die Erfindung betrifft ein Verfahren zur thermischen Steuerung mindestens einer biologischen, chemischen oder physikalisch-chemischen Reaktion in Gegenwart magnetischer Partikel, insbesondere Nanopartikel, oder magnetischer Beads *in vitro* durch Erwärmung der magnetischen Beads oder magnetischen Partikel mittels Wechselmagnetfelder auf mindestens eine bestimmte Zieltemperatur.

Die mit dem erfindungsgemäßen Verfahren thermisch steuerbare Reaktion kann allgemein eine temperaturabhängige Hybridisierungs- oder Bindungsreaktion oder eine temperaturabhängige enzymatische Reaktion umfassen. In spezielleren Ausführungsformen handelt es sich bei der temperaturabhängigen enzymatische Reaktion um eine Amplifizierung (z.B. mittels PCR), E-longation, Synthese, Ligierung oder Restriktion von Nukleinsäuren, einschließlich DNA, RNA oder Hybride oder Derivate davon.

Weitere Aspekte der Erfindung betreffen einen Reaktor zur Durchführung des Verfahrens und die Verwendung des Verfahrens bzw. des Reaktors in der Analytik und Diagnostik.

**EP 2 110 175 A1**

**Beschreibung**

Analytische und diagnostische Verfahren, welche insbesondere der Aufarbeitung einer Probe, der Isolation von Zielstrukturen- oder Molekülen, der Vervielfältigung von Zielmolekülen und dem Nachweis von Zielmolekülen dienen, haben ein breites und ständig wachsendes Spektrum von Anwendungsbereichen. Mit den Fortschritten auf den Gebieten der Gentechnik und Molekularbiologie haben in jüngerer Zeit insbesondere Verfahren, bei denen biologische und chemische Reaktionen mit Nukleinsäuren als Reaktionspartner bzw. Zielmoleküle eine wesentliche Rolle spielen, zunehmend an Bedeutung gewonnen.

Für die Vervielfältigung und den Nachweis spezifischer Sequenzen in Nukleinsäuren (RNA, DNA) kommen verschiedene Amplifikationsverfahren zum Einsatz. Das wohl gängigste Verfahren basiert auf der Polymerase-Kettenreaktion ("Polymerase Chain Reaction"; PCR), mit der eine spezifische Nukleinsäuresequenz innerhalb eines DNA-Stranges (Template) vervielfältigt werden kann. PCR kann als Teil eines Nukleinsäureassays oder als Stand-Alone-Verfahren eingesetzt werden. Hierbei werden an ein Template hybridisierte Oligonukleotidsequenzen (Primer) freie Desoxynukleotide unter Zuhilfenahme einer DNA-Polymerase angefügt. Das entstandene Amplifikationsprodukt kann nun thermisch wieder aufgespalten werden und als Template für weitere Reaktionen dienen. Auf diese Weise kann eine Zielsequenz, die in geringer Konzentration in einer DNA-Probe vorliegt, exponentiell vervielfältigt werden.

Zur Durchführung einer PCR werden kurze Sense- und Antisense-Oligonukleotide, welche die zu amplifzierende Region eingrenzen, einer Reaktionsmischung mit dem Template in niedriger Konzentration, einer thermostabilen Polymerase und einzelnen Nukleotiden beigefugt. Generell wird die Reaktionsmischung in einem dazu geeigneten abgeschlossenen Gefäß (Kapillare oder Tube) mehreren Temperaturzyklen unterzogen (Denaturierung ca. 95-100°C, Hybridisierung 40-65°C, Elongation 70-80°C, vorzugsweise ca. 72°C, abhängig von der verwendeten Polymerase). Die Temperaturzyklen werden im Allgemeinen mit einem Peltierelement als Thermoblock oder Heißluft (Lightcycler) erzeugt. Hierzu muss ein direkter Kontakt zwischen Gefäßwand und Peltierelement oder alternativ Kapillarwand und umströmender Heißluft stattfinden. Die Erreichung einer effizienten Wärmeübertragung zwischen der PCR-Reaktionsmischung und dem Heizmedium (Peltierelement, Heißluft) ist öfter problematisch, so dass die Temperaturgradienten nicht optimal realisiert werden können. Insbesondere bei automatisierten Prozessen wie vollintegrierten Diagnostiksystemen, die einen Amplifikationsschritt bsp. über PCR erfordern, ist ferner die direkte Ankopplung des Peltierelements oder einer Heißluftdüse an ein Disposable (Reagenziencartridge) erforderlich. Dazu bedarf es im Allgemeinen eines hohen mechanischen Aufwandes.

Ähnliche Probleme wie bei der Nukleinsäurevervielfältigung mittels PCR oder alternativer Amplifikationsreaktionen treten auch bei der Temperatursteuerung anderer biologischer und chemischer Reaktionen, z.B. Hybridisierungs- und Bindungsreaktionen und allgemein enzymatischer Reaktionen, auf.

Deshalb wäre es sehr vorteilhaft, wenn die Temperatur bei biologischen und chemischen Reaktionen, wie z.B. die erforderlichen Temperaturzyklen der PCR, auf einfache und effektive Weise berührungsfrei gesteuert werden könnte. Es gibt zwar grundsätzlich eine Reihe von bekannten Methoden zur berührungsfreien Temperatursteuerung chemischer und biologischer Reaktionen, z.B. die Verwendung von Mikrowellen, Infrarotlicht, Laserbestrahlung, Halogenlicht, Heißluft, Induktionserhitzung, chemische Heizmethoden etc. (siehe z.B. Biotechnology Advances 24 (2006), S. 243-284), die meisten dieser Methoden bringen jedoch einen relativ hohen apparativen Aufwand mit sich und/oder sind mit anderen Nachteilen verbunden. Insbesondere ist die Temperatursteuerung dieser Verfahren relativ ungenau, so dass zur exakten Einstellung die Verwendung von Temperatursensoren erforderlich ist, die in den Reaktionsraum oder in direktem Kontakt mit dem Reaktionsraum platziert werden müssen.

Die vorliegende Erfindung beruht auf dem überraschenden Befund, dass magnetische Beads im Reaktionsvolumen durch magnetische Wechselfelder präzise auf bestimmte Zieltemperaturen erwärmt werden können und somit eine thermische Steuerung der betreffenden Reaktionen auf bequeme und effiziente Weise erreicht werden kann, ohne dass herkömmliche Heizelemente oder Heizmedien erforderlich sind. Auch auf die Verwendung von Temperatursensoren kann bei genauer Voreinstellung der Erwärmungsbedingungen durch Art und Menge der magnetischen Partikel/Beads in vielen Fällen verzichtet werden.

Die Kombination von Nukleinsäuren mit magnetischen Beads ist grundsätzlich bekannt.

Seit einiger Zeit werden magnetische Partikel (Nanopartikel und Beads) zur Aufreinigung von Makromolekülen wie Nukleinsäuren und Proteinen, aber auch Viren, Bakterien und Säugertierzellen, verwendet. Dazu werden z.B. gegen spezifische Oberflächenstrukturen auf den Biomolekülen gerichtete Fängermoleküle (z.B. Antikörper, Haptene, Nukleinsäuren) auf der Oberfläche der magnetischen Beads immobilisiert. Alternativ kann die Oberfläche der magnetischen Beads mit speziellen Coatings wie z.B. Silanen ausgestattet werden, die eine hohe Affinität zu einer Molekülsorte aufweisen. Die Absorption der Zielmoleküle erfolgt dann unter speziellen Pufferbedingungen. Durch einen Wechsel des Puffermediums können die an die Oberfläche gebundenen Moleküle wieder gelöst (eluiert) werden.

Die Separation der Zielstruktur oder des Zielmoleküls vom Rest der Probe und deren anschließenden Reinigung erfolgt durch Anlegen eines Magnetfeldes, das die magnetischen Beads aus der Suspension in einer Region konzentriert und reversibel fixiert. Der Überstand kann dann verworfen und durch beispielsweise einen Waschpuffer ausgetauscht werden, um die auf den Beads gebundenen Zielmoleküle zu reinigen. Durch Entfernung des äußeren Magnetfeldes werden die Beads wieder resuspendiert.

Bei Nukleinsäure-basierten Aufreinigungsverfahren verwendet man meist silanisierte magnetische Beads, die eine hohe Bindungsaffinität zum positiv geladenen Rückgrat von Nukleinsäuren (DNA, RNA) aufweisen. Bei Verwendung einer hohen Konzentration von so genannten chaotropen Salzen in der Pufferlösung, die eine hydrophile Interaktion mit Ionen verringern, binden Nukleinsäuren reversibel an die Beadoberfläche. Diese Bindung wird durch eine Verringerung der Salzkonzentration oder Temperaturerhöhung wieder gelöst.

In letzter Zeit werden auch einzelsträngige Nukleinsäuren (Oligonukleotide, cDNA oder PCR-Produkte) über Linkermoleküle an die Beadoberfläche gebunden, um eine spezifische Hybridisierung mit in einer Probe befindlichen komplementären Nukleinsäuresträngen zu ermöglichen.

Neuerdings wurden Ansätze beschrieben, bei denen eine Elongation von Oligonukleotiden, die direkt oder über Spacer an Magnetbeadträger gekoppelt vorliegen, möglich ist. Liu et al. (2001) beschreiben eine PCR unter Verwendung von Primern, die an magnetische Nanopartikeln gebunden sind, wobei die PCR-Produkte direkt auf den Nanopartikeln amplifiziert werden (Biotechnol. J. 2007 Apr. 2(4):508-11).

Bead-gebundene Primer kommen unter anderem auch bei Hochdurchsatz-Screeningverfahren zum Einsatz. So wird dieses Verfahren zum Beispiel von Kojima et al. (2005) zum Screening von Transkriptionsfaktoren unter Einsatz von PCR-Amplifikation eingesetzt (Nucleic Acids Research, 2005, Bd. 33, Nr. 17).

Weiterhin ist bekannt, dass die Applikation externer Wechselmagnetfelder innerhalb bestimmter Frequenzen eine Erhitzung magnetischer Partikel in Suspensionen bewirkt. Diese Effect wird insbesondere bei der "Magnetpartikel-Hyperthermie" bei Tumorerkrankungen angewandt. Nach Injektion der Magnetpartikel in das Tumorgewebe kommt es nach Einschaltung von Wechselmagnetfeldern durch einen externen Elektromagneten zu einer örtlichen Erhitzung und Zerstörung des Tumorgewebes (U. Gneveckow, A. Jordan, R. Scholz, et al., Biomed. Technol. 50 (2005) 92; und M. Suzuki, M. Shinkai, M. Kamihira et al., Biotechnol. Appl. Biochem. 21 (1995) 1179). Dieser Effekt kann auch beim "Drug Targeting" zur gezielten Steuerung in vivo und lokalen Freisetzung von therapeutisch wirksamen Substanzen ausgenutzt werden (E. Viroonchatapan, H. Sato, M. Ueno et al., Life Sci. (1996) 58(24):2251-61.)

Die Hyperthermie fand jedoch bisher keinen Einsatz bei *in-vitro* Testsystemen, inbesondere nicht zur Steuerung von Nukleinsäure-Amplifizierungen wie z.B. PCR und ähnlichen Reaktionen.

Die Aufgabe der vorliegenden Erfindung war somit die Bereitstellung eines neuen Verfahrens zur berührungsfreien thermischen Steuerung von biologischen und chemischen Reaktionen *in vitro,* das insbesondere für die Steuerung von Nukleinsäure-Amplifizierungsreaktionen wie z.B. PCR oder Nukleinsäure-Elongationsreaktionen geeignet ist und einen geringen apparativen Aufwand erfordert.

Diese Aufgabe wird erfindungsgemäß gelöst durch die Bereitstellung des Verfahrens nach Anspruch 1 sowie des Reaktors nach Anspruch 16. Weitere Aspekte und/oder bevorzugte Ausführungsformen der Erfindung sind Gegenstand der weiteren Ansprüche.

Bei dem erfindungsgemäßen Verfahren werden biologische, chemische oder physikalisch-chemische Reaktionen *in vitro* durch Erwärmung von magnetischen Partikeln oder Beads im Reaktionsraum mit Hilfe von Wechselmagnetfeldern auf mindestens eine Zieltemperatur berührungsfrei thermisch gesteuert.

In einer bevorzugten Ausführungsform der Erfindung werden die Temperaturzyklen einer PCR-on-a-Bead durch externe Wechselmagnetfelder generiert, die zu einer Erwärmung der Partikel führen. Hierzu werden die magnetischen Beads so konzipiert, dass diese wärmestabil sind, Oligonukleotide stabil (kovalent) auf ihrer Oberfläche zu binden vermögen und durch externe Wechselmagnetfelder so anregbar sind, dass eine Erwärmung (Hyperthermie) stattfindet. Die Wärmeentwicklung wird in Abhängigkeit von den magnetischen Eigenschaften und Größe der Beads mittels eines Wechselmagnetfelds bei einer Frequenz, die vorzugsweise im 50-500 kHz-Frequenzband liegt, induziert.

Die Aufheizung der magnetischen Partikel oder Beads zur Temperatursteuerung einer PCR oder einer anderen Reaktion kann über die Curie-Temperatur der verwendeten Legierungen für die Nanopartikel, den Partikel-Gehalt pro Bead und der Anzahl der Beads pro (PCR)-Reaktionsvolumen geeicht werden. Die Erwärmung der Reaktionsmischung erfolgt über externe Wechselmagnetfelder definierter Feldstärke und Frequenz, um optimale Temperaturgradienten zu generieren. Die Abkühlung der Reaktionsmischung erfolgt passiv oder aktiv mittels Luftkühlung oder Kopplung an ein wärmeleitendes Material. Der Aufheizung und anschließenden Abkühlung liegt der so genannte magnetokalorische Effekt zugrunde, der mittlerweile in magnetischen Wärmepumpen und Kühlschränken genutzt wird.

Geeignete Feldstärken liegen typischerweise in einem Bereich von 1 bis 1000 kA/m, vorzugsweise von 10 bis 100 kA/m, bevorzugter 50 bis 100 kA/m, und sind abhängig davon, ob ferro-, para- oder superparamagnetische Partikel eingesetzt werden und vorwiegend der durch den Brown- oder den Neel-Mechanismus hervorgerufene Aufheizeffekt zum Tragen kommt. Geeignete Frequenzen liegen typischerweise in einem Bereich von 1-1000 kHz, vorzugsweise 50-500 kHz, bevorzugter in einem Bereich von 100-400 kHz. Zur Erzeugung des Wechselmagnetfelds eignet sich z.B. eine Feldstärke von 1,6 kA/m im kHz-Bereich, z.B. 300 kHz.

Die Feldstärken und Frequenzen können für den speziellen Anwendungsfall unschwer durch Routineversuche vom Fachmann optimiert werden.

Die erreichbare Temperaturerhöhung durch magnetische Beads bzw. Magnetpartikel in einem Reaktionsvolumen ergibt sich aus der Verlustleistung Qs im elektromagnetischen Feld, die eine bestimmte Menge Magnetpartikel auf das umgebende Medium (z.B. Wasser bzw. eine wässrige Lösung) übertragen. Diese Berechnung setzt voraus, dass die Verlustleistung im Wechselfeld praktisch vollständig in Wärme umgewandelt wird (durch Erfahrungswerte in der Praxis bestätigt).

Unter den typischen obigen Feldstärke- und Frequenzbedingungen liegt die spezifische Verlustleistung für Partikel von 13 nm bis 100 nm (bestimmt durch BET-Stickstoffadsorption) allgemein im Bereich von etwa 100 bis 600 W/g oder kJ/kg s.

Diese Verlustleistung kann für verschiedene Beads bzw. Magnetpartikel unschwer empirisch bestimmt werden und hinsichtlich Größe und Zusammensetzung geeignete Partikel für eine gewünschte Heizleistung können ausgewählt werden.

Für Magnetitpartikel ($Fe_3O_4$) gilt die folgende Gleichung:

$$\text{Heizrate } \Delta T = Qs\ m(Fe_3O_4)/c(H_2O)\ x\ m(H_2O)$$

Wärmekapazität $c(H_2O)$ = 4,18 kJ/kg K
Spezifische Verlustleistung von Partikeln mit 25 nm
Durchmesser: ca. 250 kJ/kg s

Ein typischer Reaktionsansatz für eine PCR oder eine andere enzymatische Reaktion unter Beteiligung von Nukleinsäuren umfasst ein Volumen von etwa 5 μl bis 1 ml, häufiger 10 μl bis 500 μl, typischerweise 50 μl bis 100 μl. Bei einem Volumen von 50 bis 100 μl (0,05 g bis 0,1 g) und einer Partikelmenge von 0,0025 g bis 0,005 g (Konzentration von 5 Gew. % in einer Standardsuspension) wie im Reaktionsansatz von Beispiel 1 ergibt sich damit z.B. eine Heizrate $\Delta T$ = 2,99 K/s.

Ändert man die Partikelkonzentration in einem Bereich von 1 bis 10 Gew.-% führt das zu einer Variation der Heizrate von 0,6 bis 6,0 K/s.

Diese Heizraten sind zur Erwärmung und Temperatursteuerung einer typischen PCR-Reaktion oder einer anderen Nukleinsäure-vervielfältigungsreaktion oder auch einer Nukleinsäureelongationsreaktion gut geeignet. Falls in bestimmten Situationen andere Heizraten erforderlich sein sollten, können diese z.B. durch Variation der Anzahl, Größe und Art der Partikel nach Wunsch eingestellt werden.

Die Bedeutung des Begriffs "Beads", insbesondere "magnetische Beads", wie hier im Zusammenhang der vorliegenden Erfindung verwendet, entspricht dem üblichen Verständnis des Fachmanns auf diesem Gebiet. Beads sind allgemein Trägerpartikel, typischerweise in einem Größenbereich von etwa 100 nm bis etwa 10 μm, vorzugsweise etwa 500 nm bis 5 μm, die funktionalisiert sein können oder nicht, vorzugsweise funktionalisiert sind, häufig von annähernd sphärischer Gestalt sind, und im Falle von magnetischen Beads in der Regel kleinere magnetische Nanopartikel umfassen, die z.B. in einer Matrix eingebettet und/oder mit einer Beschichtung versehen sind.

Diese magnetischen Nanopartikel haben typischerweise eine Größe im Bereich von etwa 10 nm bis etwa 900 nm, vorzugsweise etwa 20 nm bis 500 nm, und können ein breites Spektrum von Zusammensetzungen aufweisen, wie im Folgenden, insbesondere für den Einsatz in magnetischen Beads, näher ausgeführt. Diese magnetischen Nanopartikel können als solche zur erfindungsgemäßen thermischen Steuerung von biologischen/- chemischen Reaktionen eingesetzt werden oder als Bestandteil von größeren magnetischen Beads wie im Folgenden näher beschrieben. Auch eine Mischung verschiedener magnetischer Nanopartikel oder eine Mischung von magnetischen Nanopartikeln mit magnetischen Beads kann gegebenenfalls eingesetzt werden.

Als magnetische Beads zur Verwendung im erfindungsgemäßen Verfahren kommen grundsätzlich alle in der Literatur beschriebenen und/oder im Handel erhältlichen Beads in Betracht. Geeignete Beads können je nach der gewünschten bzw. erforderlichen Curie-Temperatur und gegebenenfalls gewünschten weiteren Oberflächeneigenschaften und Funktionalitäten aus dem breiten Spektrum von kommerziell verfügbaren Beads ausgewählt oder nach bekannten Verfahren hergestellt werden.

Die verschiedenen in der Fachliteratur (F. Schüth, et al., Angew. Chem. 2007, 119, 1242-1266) beschriebenen oder kommerziell verfügbaren (http://www.magneticmicrosphere.com) magnetischen Beads basieren auf Partikeln aus reinen Metallen wie Fe und Co, Legierungen wie $CoPt_3$, CoPt, FePt etc., bzw. oxidischen Phasen wie $\gamma$-$Fe_2O_3$, FeO, NiO und insbesondere den Spinellen $Fe_3O_4$ oder allgemein $M^{II}M^{III}_2O_4$ (M = Fe, Ni, Co, Mn, Cr, Mg, Zn etc.). Bei den meisten kommerziell erhältlichen Magnetbeads stehen die reinen Metalle und vor allem die verschiedenen Eisenoxide im Vordergrund.

Zur Herstellung der jeweiligen Partikel stehen unterschiedliche Wege zur Verfügung, wobei hier vor allem die Copräzipitation (Mitfällung), die thermische Zersetzung und/ oder Reduktion von Precursormolekülen, die Synthese in Micellen und die Hydrothermalsynthese bevorzugt sind.

Die Copräzipitation ist eine sehr einfache Methode, um vor allem Eisenoxide (z.B.: $Fe_3O4$, $\gamma$-$Fe_2O_3$) aus wässrigen $Fe^{2+}$/$Fe3^+$-Lösungen (Chloride, Nitrate, Sulfate etc.) und Basen (AlkaliHydroxide, Ammoniak-Lösung, organische Amine etc.) in hoher Ausbeute und relativ enger Größenverteilung zu erhalten. Die Reaktionsbedingungen ($Fe^{2+}$/$Fe^{3+}$-Verhältniss, pH-Wert und Ionenstärke des Mediums, Art der Salze, Temperatur: 20-90 °C, Inertgasatmosphäre) legen auch Art, Qualität und Größe der Partikel fest und müssen dementsprechend genau reproduziert werden.

Durch thermische Zersetzung von metallorganischen Vorstufen (Metallcarbonylen, Metallacetylacetonaten oder Metallcupferronaten (N-Nitrosophenylhydroxylamin-Komplexen) in hochsiedenden organischen Lösemitteln (Petrolether, Toluol, langkettige Ether etc.), die Tenside (Fettsäuren, Ölsäure, Hexadecylamine) enthalten, gelangt man in einer nicht ganz einfach zu kontrollierenden Reaktion (100 - 320 °C, Inertgasatmosphäre, Keimbildung in kurzer Zeit - Wachstum über Stunden und Tage) zu guten Ausbeuten an sehr eng verteilten (monodispersen) Partikeln von hoher Qualität. In reduzierender Atmosphäre ($H_2$) können auf diese Weise Nanopartikel der reinen Metalle bzw. Legierungen (Fe, Co, FePt, $CoPt_3$,) gewonnen werden, während oxidierende Bedingungen (Luft, $O_2$, $(CH_3)_3NO$) Metalloxide ($Fe_3O_4$, $Co_3O_4$, MnO, NiO, $Cr_2O_3$) zugänglich machen. Durch den langen Wachstumszeitraum werden allerdings meist kleinere (2-20nm) Partikel generiert.

In den (inversen) Micellen von Mikroemulsionen werden vor allem die verschiedenen Spinelle gewonnen, deren Größe (1 - 50 nm, enge Verteilung) und Form durch das Verhältnis von Wasser und "Ölphase" (vor allem Toluol) aber auch von den stabilisierenden Tensiden abhängt. Die Micellen stellen dabei einen Nanoreaktor für die Fällung von Metallsalzlösungen durch Basen dar. Solche Ansätze liefern im Allgemeinen aber nur eine geringe Ausbeute und lassen sich nur schwer aufwärts skalieren, da im Vergleich zum wässrigen Anteil immer große Mengen an organischen Lösemitteln nötig sind.

Magnetische Beads zur Verwendung im erfindungsgemäßen Verfahren weisen vorzugsweise magnetische Partikel, in der Regel Nanopartikel, auf, deren Zusammensetzung aus der Gruppe ausgewählt ist, die aus Metallen, Metalloxiden und Metalllegierungen von Fe, Ni, Co, Cu, Mn, Cr, Mg, Zn, La und Sr besteht. Je nach Anwendung können jedoch auch Partikel anderer Zusammensetzung (z.B. wie im obengenannten Stand der Technik offenbart) geeignet sein.

Die im erfindungsgemäßen Verfahren verwendeten magnetischen Beads oder magnetischen Nanopartikel umfassen ferner vorzugsweise Partikel mit einer einstellbaren Curie-Temperatur. Einige nicht-beschränkende Beispiele geeigneter Zusammensetzungen der magnetischen Partikel sind eine Cu/Ni-Legierung, $La_{1-x}Sr_xMnO_3$ ($0 \le x \le 1$), $A_{1-x}Zn_x$ + $Fe_2O_4$ (A = Ni, Mn oder Cu; $0 \le x \le 1$) und $A_xB_y$ + $Fe_{2+z}O_4$ (A, B = Ni, Mn, Mg, Ca oder Cu; x + y + z = 1) (vgl. Tabelle 1).

Durch die Wahl der Legierung der Magnetpartikel kann somit die Curie-Temperatur, bei der sich die spezifische Energieabsorptionsrate (specific power absorption rate) ändert, vorbestimmt werden. Dies kann beispielsweise zur Einstellung definierter Temperaturbereiche genutzt werden, die bei der zu steuernden Reaktion während des Erwärmungsvorgangs mit Wechselmagnetfeldern nicht überschritten werden sollen.

Tabelle 1: Magnetische Legierungen mit einstellbarer Curie-Temperatur

| Patentnummer/ Publikation | Titel | Inhaber/Autor | Kurzbeschreibung | Beschriebene Legierung | |
|---|---|---|---|---|---|
| US 6731111 B2 | Validity determination using magnetic ink having magnetic powders with different curie temperatures | Toshiba | Magnetisches Pulver zur Herstellung magnetischer Tinte mit Curie-temperaturen zwischen -50°C und 150°C und einer Partikelgröße < 10 $\mu$m. | $A_{1-x}Zn_x + Fe_2O_4$ (A=Ni/Mn/Cu) | je größer der Anteil von NiZn, desto geringer die Curietemp. |
| DE4103263A1 | Feinteiliger niederkoerzitiver Ferrit und Verfahren zu seiner Herstellung | BASF | Verfahren zur Herstellung sowie Verwendung zur Absorption elektromagnetischer Strahlung | $A_X B_Y + Fe_{2+z}O_4$ (A/B=Ni/Mn/Mg/Ca/Cu) | X+Y+Z=1 Curietemp. zwischen 18°C und 200°C; Partikelgr. <0,5 $\mu$m |
| | | | | | |
| J Biomed Mater Res B Appl Biomater. 2007 Oct 5 | T(C)-tuned biocompatible Suspension of La(0.73) Sr(0.27)MnO(3) for magnetic hyperthermia. | Prasad NK, Rathinasamy K, Panda D, Bahadur D. | Magnetpartikel zur hyperthermischen Behandlung von Krebs | $La_{1-x}Sr_X MnO_3$ | Curietemp. unter 70°C; Partikelgröße 20-100 nm |
| Biomagn Res Technol. 2004 May 8;2(1):4 | Physically synthesized Ni-Cu nanoparticles for magnetic hyperthermia | Martin Bettge, Jhunu Chatterjee Und Yousef Haik | Magnetpartikel zur hyperthermischen Behandlung von Krebs | Cu/Ni-Legierung | 33 % Cu und 67% Ni entspricht einer Curie-temp. von 0°C; 29% Cu und 71 % Ni entspricht einer Curietemp. von 41-46°C |

Die im erfindungsgemäßen Verfahren verwendeten magnetischen Beads können die magnetischen Partikel in einer natürlichen oder synthetischen Polymermatrix eingebettet aufweisen.

Solche Beads können z.B. durch Einbettung der magnetischen Partikel in natürliche (z.B. Polysacharide wie Dextran, Sepharose, Polypeptide wie Poly-L-Aspartat, Poly-L-Glutamat, Polylaktide wie Poly-P, L-laktid) oder synthetische Polymermatrices (z.B. Polyvinylalkohol, Polystyrol(derivate), Poly(meth)acrylate und -acrylamide, Polypyrrole, Polyester, Poly-s-caprolactam, etc. und Copolymere auch mit natürlichen Polymeren) oder durch anorganische Beschichtungen (z.B. $SiO_2$, Au, Carbon) gewonnen werden.

Bei der Verkapselung von Magnetpartikeln können entweder kleine Partikel (z.B. von Ferrofluiden) homogen in der Trägermatrix verteilt oder größere in Form von Kern-Schale-Partikeln aufgebaut werden. Eine weitere Möglichkeit besteht in der Infiltration von (organischen/anorganischen) porösen Materialien durch sehr kleine magnetische Nanopartikel oder Lösungen von $Fe^{2+}$ und anderen Metallionen ($Fe^{3+}$, $Co^{3+}$, $Ni^{2+}$, $Mn^{2+}$, etc.) und der anschließenden Bildung der magnetischen Partikel (z.B. Ferrite) in der Matrix. Auch solche Beads, welche die magnetischen Partikel in einer anorganischen oder organischen porösen Trägermatrix eingebettet aufweisen, können in dem Verfahren der vorliegenden Erfindung vorteilhaft verwendet werden.

Insbesondere bei Matrix-dispergierten Partikeln ("Polymerbeads") hat die Größe der Beads (bis 5 oder 10 $\mu$m) meist nichts mit der Größe der enthaltenen Magnetpartikel zu tun (oft nur einige nm), was sich durch die Messung der Magnetisierungskurve leicht bestätigen lässt (kleine Partikel zeigen eine enge Hysterese).

Sowohl Polymere als auch $SiO_2$-beschichtete Magnet-Partikel eignen sich dazu, mit unterschiedlichen Funktionalitäten ausgestattet zu werden. An die $SiO_2$-Schicht (Coating) lassen sich funktionalisierte Chlor- oder Alkoxysilane anbinden. Auf diese Weise können auch Polymerisationsinitiatoren (z.B. für die ATRP) an die Partikel gekoppelt werden, um typische Kern-Schale-Partikel mit magnetischem Kern und einer Polymerschale zu erzeugen. Diese magnetischen Beads (meist Polymerpartikel mit einpolymerisierten Eisenoxidpartikeln oder Eisenoxidpartikel mit Silicabeschichtung) können von Herstellern wie beispielsweise Bangs Laboratories Inc., bioMerieux GmbH, Chemagen Biopolymer Technology, Chemicell GmbH, Invitrogen (Dynal), MagnaMedics GmbH, Merck KGaA / EMD Chemicals, Promega, Qiagen, usw. bezogen werden. Die Größen liegen im Bereich von einigen zehn Nanometern bis etwa 3 $\mu$m und einem Magnetitgehalt zwischen 10 bis > 90%.

Solche beschichteten und gegebenenfalls funktionalisierten Beads sind zur Verwendung in der vorliegenden Erfindung besonders bevorzugt.

Je nach Aufbau, Gehalt an magnetisierbaren Partikeln (Gesamtmagnetisierbarkeit) und Funktionalisierung sind diese Beads für verschiedene Anwendungen geeignet. Die wesentlichen herkömmlichen Applikationen im Bereich Life Science und Diagnostik liegen in der manuellen und automatisierten Aufreinigung von Nukleinsäuren, der Affinitätsreinigung von rekombinanten Proteinen oder anderen Biomolekülen und der Zellseparation mit Antikörperbeschichteten Magnetbeads. Die Basispartikel mit beispielsweise Carboxy- oder Amino-Funktionalitäten können für anwenderspezifische kovalente Immobilisierungen von Liganden (z.B. Streptavidin, Protein A, Antikörper, Lectine, Enzyme wie Trypsin, Benzonase) eingesetzt werden. Auf diese Weise ist es möglich, einzelsträngige Nukleinsäuren, wie beispielsweise PCR-Produkte, synthetisierte Oligonukleotide, cDNA oder RNA auf der Beadoberfläche zu immobilisieren.

Bei einer "Elongation am Bead" durch Polymerasen gemäß einer Ausführungsform des erfindungsgemäßen Verfahrens ist eine Orientierung der einzelsträngigen Nukleinsäuren 5' nach 3' erforderlich (5'-Ende am Bead immobilisiert, während das 3' Ende frei bleibt). Dazu bietet sich die Verwendung kommerziell erhältlicher Kits an, die eine anwenderfreundliche Funktionalisierung von Oberflächen magnetischer Beads ermöglichen. Dazu zählen beispielsweise das ULS[1M]-System (Universal Linkage System) der Firma Biozym Scientific GmbH und verschiedene Linkersysteme (wie Amino-, Thiol-, Phosphat-, Biotin-, SpacerC3-Linkersysteme), wie diese bsp. von der Firma biomers.net GmbH angeboten werden.

Das erfindungsgemäße Verfahren bewirkt unter Nutzung externer Wechselmagnetfelder eine Temperatursteuerung in einer Nukleinsäure-Vervielfältigungsreaktion, wie z.B. einer PCR, oder auch anderen biologischen und chemischen Reaktionen, die eine Temperaturregulierung erfordern, in einer abgeschlossenen Kammer. Es wird dadurch möglich, Reaktionen ohne direkte Wärmeankopplung (bsp. mit Heiz- oder Peltierelementen) zu steuern und zu beeinflussen. Die Vorteile liegen vor allem in der berührungsfreien magnetischen Steuerung von Heizvorgängen innerhalb eines geschlossenen Reaktionssystems, wie beispielsweise innerhalb einer diagnostischen Cartridge. Die gleichen Magnete können zur Bewegung von magnetischen Nanopartikeln (Beads) als Carrier für Zielmoleküle beispielsweise bei einer Zellseparation, einem Probenaufschluss und einer Detektion der Zielmoleküle zum Einsatz kommen. Dies trägt maßgeblich zur Einfachheit und Kompaktheit eines diagnostischen Messsystems bei. Ferner wird die Kontaminationsgefahr aufgrund einer direkten Berührung der Cartridge mit konventionellen Methoden durch Verschleppung von Substanzen und die mechanische Abnutzung von Komponenten im Analysesystem verringert.

Einige moderne analytische Systeme sind so ausgelegt, dass im Anschluss an eine PCR-on-the-bead auch der Nachweis des denaturierten DNA-Einzelstrangs über den daran befindlichen Magnetbeadträger, der dann als Marker fungiert, stattfindet. Dieser Nachweis könnte beispielsweise über magnetoresistive Sensorelemente auf einem Mikrochip (Biochip) erfolgen, auf den dem Einzelstrang komplementäre Fängermoleküle gekoppelt sind. Hierbei erzeugt das in der Regel paramagnetische Bead unter dem Einfluss eines externen Magnetfeldes ein Streufeld, das in der Nähe des magnetoresistiven Messfeldes detektiert werden kann. Die Änderung des Widerstands des magnetoresistiven Messelements (GMR oder TMR-Sensor) ist hierbei proportional zur Anzahl der am Sensorelement gebundenen magnetischen Beads. Dies ermöglicht eine Quantifizierung der am Bead befindlichen DNA-Moleküle. Die in den meisten Fällen erforderliche Vervielfältigung der nachzuweisenden Nukleinsäuren erfolgt in der Regel durch eine PCR-Reaktion, welche bisher auf herkömmliche Weise durch ein Thermoelement (Peltier) oder Heißluft (oder Mikrowellen) mit den oben ausgeführten Nachteilen gesteuert wurde. Der Vorteil des erfindungsgemäßen Verfahrens besteht nun vor allem darin, dass alle wesentlichen Schritte, nämlich Probenaufbereitung, eine Amplifikation (PCR) der Zielnukleinsäuren am Bead und die anschließende Detektion auf einer Cartridge integriert und berührungsfrei über externe magnetische Felder (Permanent- oder Elektromagnete) gesteuert werden können.

Das erfindungsgemäße Verfahren eignet sich grundsätzlich für alle chemischen oder biologischen Reaktionen, die einer Temperatursteuerung bedürfen. Dies gilt insbesondere für diagnostische Systeme, welche der Aufarbeitung einer Probe, der Isolation von Zielstrukturen- oder Molekülen, der Vervielfältigung von Zielmolekülen und dem Nachweis von Zielmolekülen dienen. Neben der in der Molekularbiologie am häufigsten verwendeten bekannten PCR als Vervielfältigungsmethode von Nukleinsäuren sind hier routinemäßig angewandte AlternativVerfahren beispielsweise die "Strand displacement amplification", die "ligase chain reaction", die "rolling circle amplification", die "nucleic acid sequence-based amplification", die "branched DNA", die "transcription-mediated amplification", "hybrid capture" und "Invader". Gängige Methoden zur Detektierung der amplifizierten Sequenzen beinhalten den Einsatz von Fluoreszenzmarkern, Enzymen, Radioisotopen und auch magnetischen Partikeln.

Darüber hinaus können auch andere enzymatische Reaktionen, wie Nukleinsäuresynthese- und Nukleinsäureelongationsreaktionen, Restriktionsenzymverdau, die Umsetzung von Substraten, aber auch Hybridisierungs- und Bindungsinteraktionen (Biochips, Immunoassays) mit dem erfindungsgemäßen Verfahren gesteuert werden.

Ein zweiter Aspekt der vorliegenden Erfindung umfasst einen Reaktor, der zur Durchführung des erfindungsgemäßen Verfahrens geeignet ist, umfassend mindestens eine Einrichtung zur Erzeugung eines Wechselmagnetfelds (1), z.B. eine Spule, und einen Reaktionsraum (2), der die Reaktanden und die magnetischen Beads (21) umfasst. Dieser Reaktor kann darüber hinaus weitere Komponenten, z.B. eine Mess- und Steuerungseinrichtung (3) und/oder eine Kühleinrichtung (4), umfassen.

In einer spezielleren Ausführungsform handelt es sich bei dem Reaktor um einen Thermocycler für die PCR oder eine andere Nukleinsäure-Vervielfältigungsreaktion.

Ein allgemeinerer Aspekt der Erfindung betrifft ein *in vitro*-Testsystem, das einen derartigen Reaktor umfasst. Das in vitro-System oder der Reaktor kann als einen Bestandteil eine diagnostische Einheit (Cartridge) umfassen, die noch weitere Komponenten, z.B. zur Analyse und Auswertung, enthalten kann und auch als austauschbare Einheit zum einmaligen Gebrauch ausgebildet sein kann.

Eine solche diagnostische Cartridge besteht im Allgemeinen aus einem Kunststoffmaterial, in das mikrofluidische Kanäle und Reaktionskammern eingearbeitet sind, in denen chemische, biologische und physikalische Reaktionen wie beispielsweise eine Probenaufbereitung, Zielmolekülisolation und -aufreinigung, die Amplifikation der Zielmoleküle (bei Nukleinsäuren) und deren Nachweis durch Bindung an einen Microarray stattfinden können. Diese diagnostischen Cartridges werden meist als Einmalteil für die Probenanalyse verwendet und mittels eines Gerätes, in das diese eingeschoben werden, gesteuert. Die Herstellung erfolgt mithilfe von Rapid Prototyping, Stanz-, Fräs- oder Spritzgussverfahren. Beispiele diagnostischer Cartridges, die im Allgemeinen auch als Lab-on-a-Chip Systeme bezeichnet werden, sind in Patentschriften wie DE 102005029809A1 oder DE 102005049976A1 beschrieben.

FIGURENBESCHREIBUNG

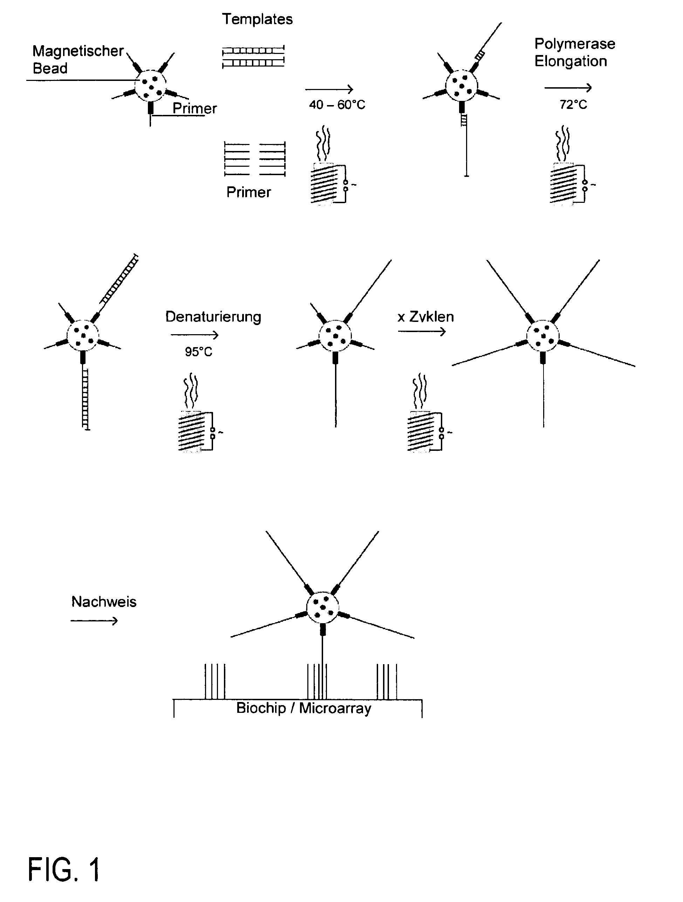

Fig. 1 zeigt eine schematische Darstellung des Ablaufs einer PCR-Reaktion mit Bead-gekoppelten Primern.

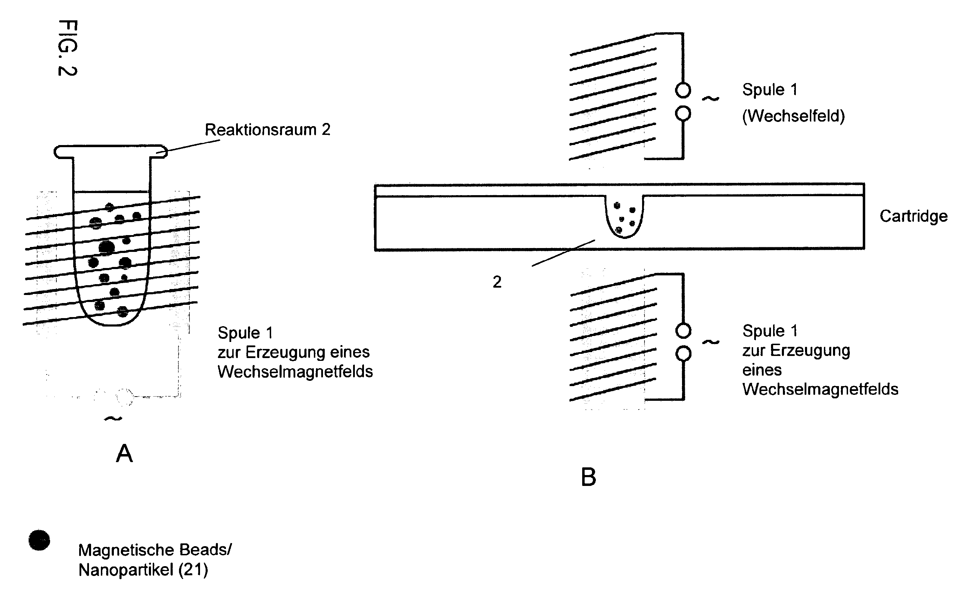

Fig. 2 zeigt schematisch zwei Varianten eines erfindungsgemäßen Reaktors zur Durchführung der Temperatursteuerung über die Erwärmung der magnetischen Beads. Feld A zeigt dabei nur die wesentlichen Komponenten zur Durchführung, unabhängig von weiteren Bestandteilen, während Feld B eine Variante zeigt, in welcher der Reaktionsraum Bestandteil einer diagnostischen Einheit (Cartridge) ist.

Das folgende Ausführungsbeispiel soll die Erfindung näher erläutern, jedoch keineswegs auf die dort offenbarten experimentellen Details beschränken.

BEISPIEL 1

Ein typischer Ansatz für eine PCR-Reaktion umfasst:

1,0 $\mu$l wässrige Lösung mit DNA-Template (circa 100 ng/$\mu$l)
2,0 $\mu$l pro Primer (Sense /Antisense) (10 $\mu$M)

1,0 µl Pfu-, Taq- oder eine andere thermostabile Polymerase (1-5 U/µl)

1,0 µl Desoxyukleotide (dNTPs) (dATP, dGTP, dCTP, dTTP je 10 mM)

5,0 µl 10-fach konzentrierte Polymerase-Pufferlösung

40,0 µl destilliertes Wasser

50,0 µl Gesamtvolumen

Zur Erzeugung des Wechselmagnetfelds wird z.B. eine Feldstärke von 1,6 kAm$^{-1}$ im kHz-Bereich, z.B. 300 kHz, gewählt.

Unter solchen Bedingungen beträgt die spezifische Verlustleistung von Magnetit-Partikeln ($Fe_3O_4$) mit 25 nm Durchmesser ca. 250 kJ/kg s.

Bei Anwendung der auf Seite 7 oben angegebenen Gleichung ergibt sich für eine Wassermenge von 50 µl (0,05 g) und eine Partikelmenge von 0,0025 g (Konzentration von 5 Gew. % in einer Standardsuspension) im obigen Ansatz eine Heizrate $\Delta T = 2,99$ K/s.

Diese Heizrate entspricht den üblichen Heizraten, die mit herkömmlichen Heizelementen für derartige PCR-Reaktionen erzielt werden, und ermöglicht die thermische Steuerung der obigen PCR-Reaktion.

Typische Temperaturen für die Temperaturzyklen einer PCR-Reaktion betragen ca. 95-100°C für die Denaturierung, 40-65°C für die Hybridisierung, 70-80°C für die Elongation (polymeraseabhängig, z.B. vorzugsweise 72°C für Taq-Polymerase). Solche Temperaturen sind mit dem erfindungsgemäßen Verfahren unschwer zu erreichen.

**Patentansprüche**

1. Verfahren zur thermischen Steuerung mindestens einer biologischen, chemischen oder physikalisch-chemischen Reaktion in Gegenwart magnetischer Partikel, insbesondere Nanopartikel, oder magnetischer Beads *in vitro* durch Erwärmung der magnetischen Beads oder magnetischen Partikel mittels Wechselmagnetfelder auf mindestens eine bestimmte Zieltemperatur.

2. Verfahren nach Anspruch 1, **dadurch gekennzeichnet, dass** die Beads funktionalisierte magnetische Beads sind.

3. Verfahren nach Anspruch 1 oder 2, **dadurch gekennzeichnet, dass** das Verfahren mehrere Zyklen von Erwärmung und Abkühlung umfasst.

4. Verfahren nach einem der Ansprüche 1 bis 3, **dadurch gekennzeichnet, dass** die biologischen, chemischen oder physikalisch-chemischen Reaktionen eine temperaturabhängige Hybridisierungs- oder Bindungsreaktion oder eine temperaturabhängige enzymatische Reaktion umfassen.

5. Verfahren nach Anspruch 4, **dadurch gekennzeichnet, dass** die temperaturabhängige enzymatische Reaktion eine Amplifizierung, Elongation, Synthese, Ligierung oder Restriktion von Nukleinsäuren, einschließlich DNA, RNA oder Hybride oder Derivate davon, umfasst.

6. Verfahren nach Anspruch 5, **dadurch gekennzeichnet, dass** die enzymatische Reaktion eine PCR-Reaktion oder eine andere Reaktion zur Vervielfältigung von Nukleinsäuren oder eine Elongationsreaktion umfasst.

7. Verfahren nach einem der Ansprüche 1-6, **dadurch gekennzeichnet, dass** die Wechselmagnetfelder eine Frequenz in einem Frequenzbereich von 1 bis 1000, insbesondere 50-500, kHz aufweisen.

8. Verfahren nach einem der Ansprüche 1-7, **dadurch gekennzeichnet, dass** die magnetischen Beads oder magnetischen Partikel Partikel mit einer einstellbaren Curie-Temperatur umfassen.

9. Verfahren nach Anspruch 8, **dadurch gekennzeichnet, dass** die Curie-Temperatur der Partikel mit einer einstellbaren Curie-Temperatur durch die Auswahl ihrer Zusammensetzung so festgelegt wird, dass ein definierter Temperaturbereich eingestellt wird, der bei der zu steuernden Reaktion während des Erwärmungsvorgangs durch Wechselmagnetfelder nicht überschritten werden soll.

10. Verfahren nach einem der Ansprüche 1-9, **dadurch gekennzeichnet, dass** die magnetischen Beads oder magnetischen Partikel magnetische Partikel umfassen bzw. darstellen, deren Zusammensetzung aus der Gruppe ausgewählt ist, die aus Metallen, Metalloxiden und Metalllegierungen von Fe, Ni, Co, Cu, Mn, Cr, Mg, Zn, La und Sr besteht.

**11.** Verfahren nach Anspruch 10, **dadurch gekennzeichnet, dass** die Zusammensetzung der magnetischen Partikel aus einer Cu/Ni-Legierung, $La_{1-x}Sr_xMnO_3$ ($0 \leq x \leq 1$), $A_{1-x}Zn_x$ + $Fe_2O_4$ (A = Ni, Mn oder Cu; $0 \leq x \leq 1$) und $A_xB_y$ + $Fe_{2+z}O_4$ (A, B = Ni, Mn, Mg, Ca oder Cu; x + y + z = 1) ausgewählt ist.

**12.** Verfahren nach einem der Ansprüche 1-11, **dadurch gekennzeichnet, dass** die magnetischen Beads die magnetischen Partikel in einer natürlichen oder synthetischen Polymermatrix eingebettet aufweisen.

**13.** Verfahren nach einem der Ansprüche 1-12, **dadurch gekennzeichnet, dass** die magnetischen Beads die magnetischen Partikel in einer anorganischen oder organischen porösen Trägermatrix eingebettet aufweisen.

**14.** Verfahren nach einem der Ansprüche 1-13, **dadurch gekennzeichnet, dass** die magnetischen Beads magnetische Partikel umfassen, die eine Silicabeschichtung oder eine andere Beschichtung aufweisen.

**15.** Verfahren nach einem der Ansprüche 1-14, **dadurch gekennzeichnet, dass** die magnetischen Partikel eine Größe im Bereich von 10 nm bis 500 nm und die magnetischen Beads eine Größe im Bereich von 500 nm bis 5 μm aufweisen.

**16.** Reaktor, der zur Durchführung des Verfahrens nach einem der Ansprüche 1-15 geeignet ist, umfassend eine Einrichtung zur Erzeugung eines Wechselmagnetfelds (1) und einen Reaktionsraum (2), der die Reaktanden und die magnetischen Beads (21) umfasst.

**17.** Reaktor nach Anspruch 16, der ferner eine Mess- und Steuerungseinrichtung (3) und gegebenenfalls eine Kühleinrichtung (4) umfasst.

**18.** Reaktor nach Anspruch 16 oder 17, **dadurch gekennzeichnet, das** sich um einen Thermocycler für die PCR oder eine analoge Vorrichtung für eine andere Nukleinsäure-Vervielfältigungsreaktion handelt.

**19.** Reaktor nach einem der Ansprüche 16-18, umfassend eine diagnostische Cartridge.

**20.** In vitro-Testsystem, umfassend einen Reaktor nach einem der Ansprüche 16-19.

**21.** In vitro-Testsystem nach Anspruch 20, umfassend eine diagnostische Cartridge.

**22.** Verwendung des Verfahrens nach einem der Ansprüche 1-15 oder des Reaktors nach einem der Ansprüche 16-19 in der Analytik und Diagnostik.

**23.** Verwendung nach Anspruch 22, welche die Aufarbeitung oder Reinigung einer Probe, die Isolation von Zielstrukturen und/oder Zielmolekülen, und die Vervielfältigung und/oder Identifizierung von Zielmolekülen umfasst.

**24.** Verwendung magnetischer, vorzugsweise funktionalisierter, Beads oder magnetischer Nanopartikel zur thermischen Steuerung mindestens einer biologischen, chemischen oder physikalisch-chemischen Reaktion *in vitro*.

Templates

Magnetischer Bead

Polymerase Elongation

Primer

40 – 60°C

72°C

Primer

Denaturierung

x Zyklen

95°C

Nachweis

Biochip / Microarray

# FIG. 1

EP 2 110 175 A1

FIG. 2

Reaktionsraum 2

Spule 1
zur Erzeugung eines
Wechselmagnetfelds

A

Spule 1
(Wechselfeld)

Cartridge

2

Spule 1
zur Erzeugung
eines
Wechselmagnetfelds

B

● Magnetische Beads/
Nanopartikel (21)

**Europäisches Patentamt**

# EUROPÄISCHER RECHERCHENBERICHT

**Nummer der Anmeldung**

EP 08 00 5370

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 198 00 294 A1 (MUELLER SCHULTE DETLEF DR [DE]) 8. Juli 1999 (1999-07-08) <br> * Spalte 1, Zeile 3 - Zeile 7 * <br> * Spalte 2, Zeile 55 - Spalte 5, Zeile 37 * <br> * Spalte 5, Zeile 65 - Spalte 6, Zeile 9 * <br> * Spalte 8, Zeile 44 - Spalte 9, Zeile 4 * <br> * Spalte 10, Zeile 8 - Spalte 11, Zeile 1; Beispiel 1 * <br> ----- | 1-12, 14-24 | INV. <br> B01L7/00 <br> H01F1/36 <br> H01F1/37 <br> H05B6/02 <br> H05B6/10 |
| X | WO 2008/027090 A (UNIV CALIFORNIA [US]; SAILOR MICHAEL J [US]; PARK JI-HO [US]; DERFUS A) 6. März 2008 (2008-03-06) <br> * Seite 1, Zeile 27 - Seite 2, Zeile 7 * <br> * Seite 2, Zeile 29 - Zeile 32 * <br> * Seite 8, Zeile 1 - Zeile 13 * <br> * Seite 9, Zeile 8 - Zeile 29; Abbildung 9b * <br> ----- | 1-7,10, 13,15-24 | |
| X | JP 2006 061031 A (HITACHI MAXELL) 9. März 2006 (2006-03-09) <br> <br> * Zusammenfassung * <br> * Absatz [0007] * <br> * Absatz [0017] * <br> * Absatz [0036] * <br> ----- | 1-8, 10-12, 14,16-24 | RECHERCHIERTE SACHGEBIETE (IPC) <br> B01L <br> H01F <br> H05B |
| X | WO 2004/052527 A (KNOELL HANS FORSCHUNG EV [DE]; FRIEDRICH SCHILLER UNI VERSITA [DE]; MU) 24. Juni 2004 (2004-06-24) <br> * Seite 10, Zeile 29 - Seite 11, Zeile 13 * <br> * Seite 12, Zeile 12 - Zeile 14; Beispiel 2 * <br> ----- <br> -/-- | 1-6, 16-18, 20,22-24 | |

Der vorliegende Recherchenbericht wurde für alle Patentansprüche erstellt

| Recherchenort | Abschlußdatum der Recherche | Prüfer |
|---|---|---|
| München | 24. Juli 2008 | Marti, Pedro |

EPO FORM 1503 03.82 (P04C03)

Europäisches
Patentamt

**EUROPÄISCHER RECHERCHENBERICHT**

Nummer der Anmeldung

EP 08 00 5370

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | JP 2003 028880 A (ALOKA CO LTD) 29. Januar 2003 (2003-01-29)<br><br>* Zusammenfassung *<br>----- | 1,2,4,5, 8-10,14, 16,17, 20,22,24 | |
| X | US 2006/226832 A1 (IKEDA TAKASHI [JP] ET AL) 12. Oktober 2006 (2006-10-12)<br><br>* Absatz [0020] *<br>* Absatz [0023] - Absatz [0025] *<br>* Absatz [0033] - Absatz [0039] *<br>* Absatz [0041] - Absatz [0043] *<br>----- | 1,2,4,5, 10,16, 17,20, 22-24 | |
| X | WO 03/054102 A (SUS TECH GMBH & CO KG [DE]; SAUER HANS-MARTIN [DE]; CURA ELISABETH [DE] 3. Juli 2003 (2003-07-03) * das ganze Dokument *<br>----- | 1,8-11, 16 | |
| A | US 2005/249817 A1 (HAIK YOUSEF [US] ET AL) 10. November 2005 (2005-11-10) * das ganze Dokument *<br>----- | 1-24 | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 24. Juli 2008 | Marti, Pedro |

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**                    EP 08 00 5370

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-07-2008

| Im Recherchenbericht angeführtes Patentdokument | Datum der Veröffentlichung | Mitglied(er) der Patentfamilie | | Datum der Veröffentlichung |
|---|---|---|---|---|
| DE 19800294 A1 | 08-07-1999 | KEINE | | |
| WO 2008027090 A | 06-03-2008 | KEINE | | |
| JP 2006061031 A | 09-03-2006 | KEINE | | |
| WO 2004052527 A | 24-06-2004 | AU | 2003294652 A1 | 30-06-2004 |
| | | DE | 10258398 A1 | 12-08-2004 |
| JP 2003028880 A | 29-01-2003 | KEINE | | |
| US 2006226832 A1 | 12-10-2006 | CN | 1947016 A | 11-04-2007 |
| | | JP | 2005315677 A | 10-11-2005 |
| | | WO | 2005106480 A1 | 10-11-2005 |
| WO 03054102 A | 03-07-2003 | AT | 318295 T | 15-03-2006 |
| | | AU | 2002363875 A1 | 09-07-2003 |
| | | DE | 10163399 A1 | 10-07-2003 |
| | | EP | 1456318 A1 | 15-09-2004 |
| | | ES | 2259393 T3 | 01-10-2006 |
| | | JP | 2005534720 T | 17-11-2005 |
| US 2005249817 A1 | 10-11-2005 | KEINE | | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6731111 B2
- DE 4103263 A1
- DE 102005029809 A1
- DE 102005049976 A1


**In der Beschreibung aufgeführte Nicht-Patentliteratur**

- *Biotechnology Advances,* 2006, vol. 24, 243-284
- *Biotechnol. J.,* April 2007, vol. 2 (4), 508-11
- **Kojima et al.** zum Screening von Transkriptionsfaktoren unter Einsatz von PCR-Amplifikation eingesetzt. *Nucleic Acids Research,* 2005, vol. 33 (17
- **U. Gneveckow ; A. Jordan ; R. Scholz et al.** *Biomed. Technol.,* 1995, vol. 50, 92
- **M. Suzuki ; M. Shinkai ; M. Kamihira et al.** *Biotechnol. Appl. Biochem.,* 1995, vol. 21, 1179
- **E. Viroonchatapan ; H. Sato ; M. Ueno et al.** *Life Sci.,* 1996, vol. 58 (24), 2251-61
- **F. Schüth et al.** *Angew. Chem.,* 2007, vol. 119, 1242-1266